(19) Europäisches Patentamt / European Patent Office / Office européen des brevets

(11) **EP 4 339 611 A1**

(12) **EUROPEAN PATENT APPLICATION**

(43) Date of publication:
**20.03.2024 Bulletin 2024/12**

(21) Application number: **23190405.3**

(22) Date of filing: **08.08.2023**

(51) International Patent Classification (IPC):
**G01N 33/18** (2006.01)     **C12M 1/34** (2006.01)
**C12M 1/36** (2006.01)

(52) Cooperative Patent Classification (CPC):
**G01N 33/1806; B09B 3/60; B09B 2101/70;
B09B 2101/75**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.09.2022 CN 202211133666**

(71) Applicant: **Nova Skantek (Hunan) Environ Energy
Co., Ltd**
**410100 Changsha Hunan (CN)**

(72) Inventors:
- **LI, Chao**
  **Changsha, Hunan, 410100 (CN)**
- **XUE, Hanguang**
  **Changsha, Hunan, 410100 (CN)**
- **FAN, Changzheng**
  **Changsha, Hunan, 410100 (CN)**

(74) Representative: **Alatis**
**3, rue Paul Escudier**
**75009 Paris (FR)**

(54) **TEST SYSTEM AND TEST METHOD FOR AEROBIC BIODEGRADABILITY**

(57)    The present disclosure provides a test system and a test method for aerobic biodegradability. The system includes: an oxygen supply unit (1) configured to store oxygen required for aerobic biodegradation; a respirometer cells unit (2) configured to introduce the oxygen from the oxygen supply unit (1) to an aerobic fermentation unit (3) through a gas inlet pipe (6b); the respirometer cells unit is further connected to the oxygen supply unit through a gas pipe (6a), and is further configured to calculate a volume of consumed oxygen that is introduced into the aerobic fermentation unit (3) due to a negative pressure; a data processing unit (5) is configured to conduct data acquisition during the aerobic biodegradation, and calculate a biodegradation rate of the aerobic biodegradation according to the volume of consumed oxygen; the aerobic fermentation unit includes a fermentation reactor and a two-stage in-situ adsorption module that are arranged inside a constant-temperature water bath. The two-stage in-situ adsorption module includes a solid-state adsorption unit and a liquid-state adsorption unit.ol kkkkkid-state adsorption unit and a liquid-state adsorption u

FIG.2

## Description

### TECHNICAL FIELD

The present disclosure relates to the technical field of scientific instrument specifically designed to determine the biodegradability, in particular to a test system and a test method for aerobic biodegradability.

### BACKGROUND

Among the waste organic pollutants, some complex organic compounds can be transformed into simple organic or inorganic compounds through a series of chemical reactions such as oxidation, reduction, hydrolysis, dehydrogenation, and denitrification under the catalysis of natural environmental enzymes. However, a considerable number of complex organic compounds are difficult to degrade in the natural environment, posing a huge threat to human beings and ecology. In the study of biodegradation of organic pollutants, it is highly important to know how far the biodegradation of organic pollutants can proceed. Therefore, accurate measurement of microbial aerobic degradability of organic pollutants is of great significance for the study of treating organic wastes by aerobic fermentation.

In recent years, there has been a growing demand for biodegradability of materials such as organic solid wastes and plastics. Scientific researches have been conducted on the migration and transformation laws of such compounds under aerobic conditions and their impact on the environment through many different methods and theories. Although it has been possible to quantitatively test the biodegradation performance of thousands of different compounds under aerobic conditions in the past 30 years, it is difficult to compare and analyze the biodegradation performance data in different literatures. This is mainly due to the variety of test conditions and test methods used. For example, the inoculum, nutrient mixture, liquid volume and headspace capacity, **pH,** headspace pressure and degree of material mixing may all vary. These differences are especially pronounced for compounds tested under aerobic conditions.

Aerobic biodegradability is a measured of how organic matter is decomposed by aerobic bacteria. It is usually determined by measuring the oxygen volume consumed by the microorganisms, or by the composition of intermediates and final products of different microbial reactions. The conventional technique is to measure the headspace pressure in a closed bottle using a digital pressure sensor, and to measure the amount of dissolved $CO_2$ by measuring the amount of inorganic carbon (IC) with a total organic carbon (TOC) analyzer, then to calculate the amount of released $CO_2$ during degradation. This method requires regular manual operations and has large errors.

Liquid displacement and buoyancy is a commonly-used ultra-low gas measurement method in researches on anaerobic digestion of the environmental field. The method can measure more accurately and has been used for the measurement of positive-pressure gas production. However, this method allows for the continuous supply of air or oxygen, whereas classical measurement methods are few used for the of negative-pressure measurement such as oxygen consumption in aerobic biodegradation.

In addition, for the test system of aerobic biodegradability, the efficient adsorption and fixation of carbon dioxide gas produced by fermentation is a key to the accuracy of the test, which is also a core component of the entire standardized system. Generally, the biodegradation performance test is conducted by connecting a fermenter hose to a separately-designed alkaline solution adsorption device. In recent years, some researchers and companies have proposed a test method for the biodegradability of compost aiming at the biodegradation of organic matters. For example, Chinese utility model patent CN201721917449.X disclosed a microbial degradation respirometer. The device can measure gas consumption to determine oxygen consumption rate by a bubble counting method, showing the growing use of automation in instrumentation. However, the device has a low absorption efficiency of heterotopic alkaline solution absorber, and may generate bubbles with different sizes (volumes) at different gas flow rates, leading to large measurement errors. Chinese utility model patent CN202120574548.2 disclosed a fully-automatic biochemical oxygen demand measuring instrument. The instrument implements the combination of software and hardware based on the principle of drainage method to realize automatic measurement. However, no condenser is installed in front of an adsorption unit mentioned in the patent, causing a large amount of water vapor in a reactor to enter the adsorption unit during digestion, thus affecting the efficiency of the compost degradation.

### SUMMARY

It is an object of the present invention to mitigate the above problems, and to improve the efficiency of carbon dioxide adsorption, and the loss of water during the reaction, resulting in a decrease in the speed of biochemical reactions. In addition, the water vapor in the reaction rises and cools, and part of it will flow back to the adsorption unit within the reactor, then the alkaline solution is diluted, driving the utilization efficiency decrease.

According to a first aspect, an example of the present disclosure provides a test system for aerobic biodegradability, including: an oxygen supply unit, an aerobic fermentation unit, a respirometer cells unit, and a data processing unit, where

the oxygen supply unit is configured to store oxygen required for aerobic biodegradation;
the respirometer cells unit is connected to the aerobic fermentation unit through a gas inlet pipe, and is configured to introduce the oxygen from the oxygen supply unit to the aerobic fermentation unit; a one-way valve is provided on the gas inlet pipe to control a gas flow direction; the respirometer cells unit is further connected to the oxygen supply unit through a gas pipe, and is further configured to calculate a volume of consumed oxygen that is introduced into the aerobic fermentation unit due to a negative pressure;
the aerobic fermentation unit uses the oxygen introduced to improve the reaction mass transfer and carry out degradation reactions;
the data processing unit is configured to acquire data during the aerobic biodegradation, and calculate a biodegradation rate of the aerobic biodegradation according to the volume of consumed oxygen;
the aerobic fermentation unit includes a constant-temperature water bath, a fermentation reactor, and a two-stage *in-situ* adsorption module. The fermentation reactor and the two-stage *in-situ* adsorption module are arranged in the water bath by circulating water through it from an external temperature controller;
the fermentation reactor is configured to conduct degradation, and the degradation refers to that a reaction material is degraded by the aerobic biodegradation; and
the two-stage *in-situ* adsorption module is configured to adsorb carbon dioxide produced by the degradation of samples in the fermentation reactor.

In the example of the present disclosure, the test system for aerobic biodegradability has a high degree of automation, simple operation, and convenient use, and can process multiple samples by multiple channels working in parallel, with a high test efficiency; oxygen enters the aerobic fermentation system through a gas flow meter to promote the degradation of the material, the carbon dioxide produced by the reaction is adsorbed, that causes the negative pressure in the reactor to ensure the external oxygen enter the reactor; the system improves the accuracy of data, and the automated processing greatly saves manpower.

In a first implementation ofthe first aspect, the aerobic fermentation unit further includes a stirring device, and the stirring device is a mechanical stirring bar or a magnetic stirrer.

The mechanical stirring bar or the magnetic stirrer is arranged in the fermentation reactor, which is configured to promote the contact between aerobic microorganisms in the fermentation reactor and oxygen, increase dissolved oxygen, and speed up the mixing of materials to realize mass transfer and heat transfer, thereby facilitating the degradation.

In a second implementation of the first aspect, the two-stage *in-situ* adsorption module includes a solid-state adsorption unit and a liquid-state adsorption unit;

the solid-state adsorption unit is arranged below an inner mouth of the fermentation reactor and fixed by a bottle mouth, and is configured to conduct primary adsorption of the carbon dioxide produced by the degradation; and
the liquid-state adsorption unit is arranged above a mouth ofthe fermentation reactor and fixed by tightening with a threaded mouth, and is configured to adsorb the carbon dioxide not adsorbed by the solid-state adsorption unit.

With reference to the second implementation of the first aspect, in a third implementation ofthe first aspect,

the solid-state adsorption unit includes a vented container, and a grid hole structure is provided on a side wall ofthe cavity to facilitate penetration ofthe carbon dioxide; and
the liquid-state adsorption unit includes an inverted-conical slope reflux structure outside a top cover, and the inverted-conical slope reflux structure is configured to return condensed water vapor in the fermentation reactor to the fermentation reactor.

With reference to the second implementation of the first aspect, in a fourth implementation of the first aspect, the liquid-state adsorption unit includes a double-layer cavity formed by an inverted-conical slope outside a top cover and the top cover, and a fluorinated coolant is injected into the double-layer cavity to prevent the loss of moisture in a material.

With reference to the second implementation ofthe first aspect, in a fifth implementation ofthe first aspect, the liquid-state adsorption unit further includes a top liquid injection hole and a bottom liquid discharge port; the top liquid injection hole is provided with a protruding column structure on a top cover surface, and is configured to inject an alkaline solution into the liquid-state adsorption unit; and the bottom liquid discharge port is arranged at a lowest point of a bottom level ofthe liquid-state adsorption unit, and is configured to drain the alkaline solution in the liquid-state adsorption unit.

With reference to the first aspect, in a sixth implementation ofthe first aspect, the system further includes the data processing unit; the data processing unit includes a temperature sensor and a pressure sensor, and the temperature sensor and the pressure sensor are connected in the respirometer cells unit and are configured to conduct conversion into a volume of an oxygen-consuming gas under standard conditions.

According to a second aspect, an example of the present disclosure provides a test method for aerobic biodegradability, applied to the test system for aerobic biodegradability, and including the following steps:

receiving oxygen from the oxygen supply unit, determining an oxygen concentration, and calculating a volume of consumed oxygen through the respirometer cells unit;

conducting the degradation reaction of organic matter by the action of introduced oxygen and aerobic microorganisms;

calculating a $CO_2$ concentration produced by the degradation according to the volume of consumed oxygen and the oxygen concentration; and

calculating a biodegradation rate according to the $CO_2$ concentration; where the biodegradation rate represents the aerobic biodegradability.

In combination with the second aspect, in a first implementation of the second aspect, a process of calculating a biodegradation rate according to the $CO_2$ concentration includes:

calculating a theoretical amount of evolved carbon dioxide (ThCO2) for the degradation at 0°C under a standard atmospheric pressure, expressed in g, according to the following formula:

$$ThCO2 = Mvs x Cvs x 44/12$$

in the formula:

*Mvs* indicates a volatile solid in a test sample added into a compost container at the beginning of a test, in g;
*Cvs* indicates a ratio of TOC to the volatile solid in the test sample, in g/g;
44 and 12 indicate a relative molecular mass of carbon dioxide and a relative atomic mass of carbon;
*Mvs* is the volatile solid of biomass put into the fermentation reactor; compared with the total solid (TS), *Mvs* mainly characterizes the content of organic matter and helps better measure the degree of degradation of the sample;

calculating the biodegradation rate Dt(%) of the aerobic biodegradation according to the following formula:

$$Dt = [Vsr - VsB] * z: \backslash ! ThCO2 x 100$$

in the formula:

*(CO2)T* indicates an amount of carbon dioxide released equivalent to a cumulative consumption of oxygen by each compost fermentation reactor containing a mixing sample, in g;
*(CO2)B* indicates an average of carbon dioxide released equivalent to a cumulative consumption of oxygen by a blank fermentation reactor, in g;
*ThCO2* indicates a theoretical amount of evolved carbon dioxide produced by the test sample, in g;
*(CO2)T* and *(CO2)B* represent the cumulative amount of carbon dioxide emitted by each compost container containing the mixing sample, and the average of the cumulative amount of carbon dioxide emitted by the blank container, respectively;

calculating a volume of accumulated gas through the respirometer cells unit:

$$Vs = Efr P'' lw * VMc$$

in the formula:

*Vs* indicates an amount of carbon dioxide released equivalent to a volume of gas consumed by each reaction reactor at 0°C under a standard atmospheric pressure, in L;

*frr* indicates factors considering ambient temperature and air pressure;

$J_W$ indicates a factor considering a water vapor content in the gas;

*VMc* indicates a theoretical volume of the cell chamber, which is the gas volume pass through under a certain flow rate with the chamber flipping once;

obtaining the following formula according to the ideal gas equation of state:

$$frr = Vg/Vgas = \{(Pgas *(Ts+20)]/(Ps *Tgas\}$$

according to the Dalton's Law of Partial Pressure and the Antoine equation, obtaining the factor (**L**) of the water vapor content in the gas:

$$t > 1-[101.32*1 os.om1-m0.63!(233.426+ti]]l(160*PJ$$

calculating the amount of carbon dioxide (g) released by each compost fermentation reactor containing the mixing sample according to the following formula:

$$(CO2)T=44 *(Vs)r/22.4$$

calculating the average amount of carbon dioxide (g) released from the blank fermentation reactor according to the following formula:

$$(CO2)s=44 *(Vs)s/22.4$$

calculating the biodegradation rate (%) of the aerobic biodegradation according to the following formula:

$$Dt=[Vsr-VssJ*z: \backslash ThCO2x100$$

in the formula:

*Vsr* indicates an amount of carbon dioxide released equivalent to a volume of gas consumed by each test sample-containing reaction reactor at 0°C under a standard atmospheric pressure, in L; and

*Vss* indicates an average amount of carbon dioxide released equivalent to an average volume of gas consumed by each blank reaction reactor at 0°C under a standard atmospheric pressure, in L.

In a second implementation of the second aspect, the method further includes the following steps:

S1, inoculum pretreatment: screening the inoculum according to a particle size of less than 1.0 cm x 1.0 cm;

S2, determining total organic carbon (TOC) of a test sample and a reference material, and expressing the TOC in grams of the TOC per gram of a volatile solid; and pretreating the test sample and the reference material into a predetermined shape with maximum surface area of any individual piece that shall be about 1.5 cm x 1.5 cm;

S3, mixing the test sample, adjusting the moisture content of the mixture, and introducing the mixture into the aerobic fermentation unit;

S4, measuring a volume of consumed oxygen by the respirometer cells unit, and determining an amount of carbon dioxide released; and

S5, calculating the biodegradation rate according to the measured release amount of carbon dioxide and the theoretical amount of evolved carbon dioxide; where the biodegradation rate represents the aerobic biodegradability.

According to a third aspect, an example of the present disclosure provides a control computing unit, including: a memory and a processor; where the memory and the processor are connected in communication with each other, and computer instructions are stored in the memory; an in-house developed software program is then used together with the respirometer cells in order to record and display, the processor executes the computer instructions (including but not limited to calculation and calibration), thereby executing the test method for aerobic biodegradability in the second aspect or any implementation of the second aspect.

According to a fourth aspect, an embodiment of the present disclosure provides a computer-readable storage medium; where the computer-readable storage medium stores the computer instructions; the computer instructions are used to make the computer execute the test method for aerobic biodegradability in the second aspect or any implementation of the second aspect.

In the example of the present disclosure, the measurements for aerobic biodegradability with real-time pressure, temperature, and moisture compensation, ensuring accurate and reliable data acquisition; The pressure inside the reactor decreased, after the formed carbon dioxide captured by alkaline absorbent material, drives oxygen gas go through the respirometer cells unit to fermentation system for improving mass transfer and heat transfer; the system automatically performs the calculations as the experiment progresses. The mechanical stirring bar or the magnetic stirrer is arranged in the fermentation reactor, which is configured to promote the contact between microorganisms and oxygen gas, thereby facilitating the degradation.

The two-stage *in-situ* adsorption module improves an adsorption efficiency of carbon dioxide, effectively prevents the evaporation of water in the sample, and avoids the unsmooth degradation of the material; meanwhile, the water vapor does not return to dilute the alkaline solution after cooling, thereby improving a utilization efficiency of the alkaline solution, without frequently replacing the alkaline solution.

*Mvs* is a volatile solid of biomass put into the compost container. Compared with a total solid (TS), *Mvs* mainly characterizes a content of organic matters and can better measure a degree of degradation of the degraded products.

## BRIEF DESCRIPTION OF THE DRAWINGS

Below, the drawing is described in more detail. While the specification has described in detail certain exemplary embodiments, it will be appreciated that those skilled in the art, upon attaining an understanding of the foregoing, may readily conceive of alterations to, variations of, and equivalents to these embodiments.

FIG. 1 shows a schematic diagram of a test system for aerobic biodegradability according to an example of the present disclosure;
FIG. 2 shows a structural diagram of a two-stage adsorption module of the test system for aerobic biodegradability according to a preferred example of the present disclosure;
FIG. 3 shows a schematic diagram of results of an adsorption effect of the two-stage adsorption module of the test system for aerobic biodegradability according to a preferred example of the present disclosure;
FIG. 4 shows a biodegradation curve of a test method for aerobic biodegradability according to a preferred example of the present disclosure; and
FIG. 5 shows a flowchart of the test method for aerobic biodegradability according to a preferred example of the present disclosure.

## DETAILED DESCRIPTION OF THE EMBODIMENTS

In order to make the objectives, technical solutions and advantages of the embodiments of the present disclosure clearer, the technical solutions in the embodiments of the present disclosure will be clearly and completely described below in conjunction with the drawings in the embodiments of the present disclosure. Obviously, the described embodiments are some, rather than all of the embodiments of the present disclosure. All other examples obtained by those skilled in the art based on the examples of the present disclosure without creative efforts shall fall within the protection scope of the present disclosure.

In this example, a test system for aerobic biodegradability is provided. As used below, the term "module" may implement the combination of software and/or hardware having predetermined functions. Although the system described in the following embodiments is preferably implemented by software, implementation by hardware or the combination of the software and the hardware is also possible and may be conceived.

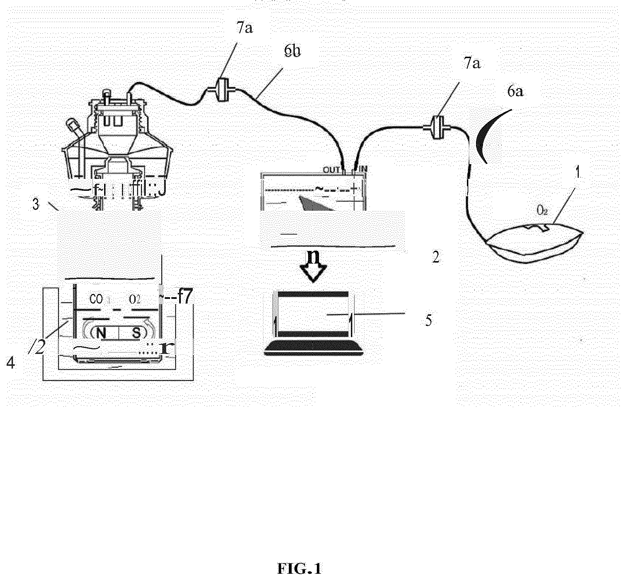

Therefore, aiming at the above problems, the present disclosure provides a test system for aerobic biodegradability, as shown in FIG. 1. The system includes: an oxygen supply unit 1, a respirometer cells unit 2, an aerobic fermentation unit 3, and a data processing unit 5; where the oxygen supply unit 1 is configured to store oxygen required for aerobic biodegradation; the respirometer cells unit 2 is connected to the oxygen supply unit 1 through a gas pipe 6a, and is configured to calculate a volume of consumed oxygen introduced into the aerobic fermentation unit 3 due to a negative pressure; a one-way valve 7a is provided on the gas pipe 6a. The respirometer cells unit 2 is connected to the aerobic fermentation unit 3 through a gas inlet pipe 6b for introducing the oxygen from the oxygen supply unit 1 into the aerobic fermentation unit 3; the one-way valve 7a is provided on the gas inlet pipe 6b and is configured to control agas flow direction.

Oxygen is introduced into the aerobic fermentation unit 3 to conduct aerobic degradation on degradation products; the data processing unit 5 is configured to conduct data acquisition during the aerobic degradation, and calculates a biodegradation rate of the aerobic degradation according to the volume of consumed oxygen; the aerobic fermentation unit 3 includes a constant-temperature water bath 4, a fermentation reactor, and a two-stage *in-situ* adsorption module. The fermentation reactor and the two-stage *in-situ* adsorption module are arranged in the constant-temperature water bath 4; the fermentation reactor is configured to conduct degradation, and the degradation is aerobic biodegradation of a reaction material; the two-stage *in-situ* adsorption module is configured to adsorb carbon dioxide produced by the degradation of samples in the fermentation reactor. The specific structures of the fermentation reactor and the two-stage *in-situ* adsorption module are detailed below.

In practical applications, the test system for aerobic biodegradability provided in this example further includes the data processing unit 5; the data processing unit 5 includes a temperature sensor and a pressure sensor, and the temperature sensor and the pressure sensor are connected in the gas metering unit and are configured to conduct conversion into a volume of an oxygen-consuming gas under standard conditions. Moreover, much high data quality can be obtained which can be used to extract kinetic information of the biodegradation process.

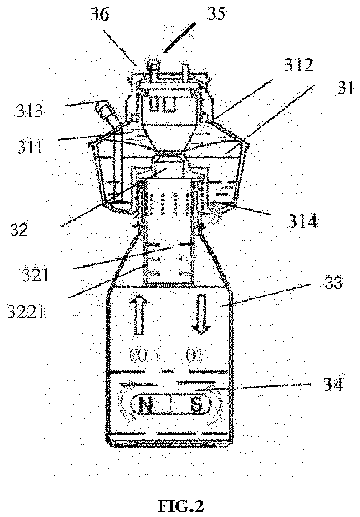

In another specific example, as shown in FIG. 2, the aerobic fermentation unit 3 further includes a stirring device 34, and the stirring device 34 is a mechanical stirring bar or a magnetic stirrer; the mechanical stirring bar or the magnetic stirrer is arranged in the fermentation reactor 33, which is configured to increase mass transfer between the sample and aerobic microorganisms within the fermentation reactor 33, providing sufficient oxygen transfer capability to support desired cell mass concentration with minimum energy,

Specifically, the two-stage *in-situ* adsorption module includes a solid-state adsorption unit 32 and a liquid-state adsorption unit 31; the solid-state adsorption unit 32 is arranged below an inner mouth of the fermentation reactor 33 and fixed by a bottle mouth, and is configured to conduct primary adsorption of the carbon dioxide produced by the degradation; and the liquid-state adsorption unit 31 is arranged above a mouth of the fermentation reactor 33 and fixed by tightening with a threaded mouth, and is configured to adsorb the carbon dioxide not adsorbed by the solid-state adsorption unit 32. The solid-state adsorption unit 32 includes a vented container 321, and a grid hole structure 3221 is provided on a side wall of the cavity to facilitate penetration of the carbon dioxide.

The liquid-state adsorption unit 31 includes an inverted-conical slope 311 inside a top cover, and the slope is configured to reflux condensed water vapor into the fermentation reactor 33; the liquid-state adsorption unit 31 further includes a double-layered cavity 312 formed by the inverted-conical slope 311 inside of the top cover and the top cover, and the liquid for cooling is injected into the double-layered cavity 312. Due to desirable chemical inertness, electrical insulation performance, and thermal conductivity of the fluorinated coolant, electronic fluorinated liquid has a high boiling point, and its volatilization with time is relatively small, thus greatly improving a condensation effect of water vapor in the fermentation reactor and effectively preventing the loss of moisture in a material. The liquid-state adsorption unit 31 further includes a top liquid injection hole 313 and a bottom liquid discharge port 314. The top liquid injection hole 313 is provided with a protruding column structure on a top cover surface, and is configured to inject a lye into the liquid-state adsorption unit 31; and the bottom liquid discharge port 314 is arranged at a lowest point of a bottom level of the liquid-state adsorption unit 31, and is configured to drain the lye in the liquid-state adsorption unit 31.

Since a reaction temperature in the aerobic respiration is required to be controlled at 58°C±2°C, water in the reaction material is easily evaporated, and then absorbed by a solid alkaline or condensed and returned to the liquid-state adsorption unit, causing dilution of the alkaline solution to affect a CO2 adsorption efficiency, and at the same time causing water loss in the reaction material to affect the biodegradation effect. In order to solve this problem, a condensation and reflux structure is added into the design: the inverted-conical slope 311 outside a top cover and the top cover forma double-layer cavity 312, into which the liquid for cooling is injected. Due to its well chemical inertness, electrical insulation performance, and thermal conductivity, the liquid for cooling has a high boiling point and its volatilization over time is relatively small. Therefore, a water vapor condensation effect in the fermentation reactor is greatly improved, thereby effectively preventing the loss of water in the material without affecting an adsorption efficiency, so as to reduce a power source of the cycle reaction. In this example, the liquid and solid two-stage adsorption device greatly improves the adsorption efficiency of CO2, and effectively forms a negative pressure to provide a power source, thereby ensuring a stable and continuous aerobic reaction.

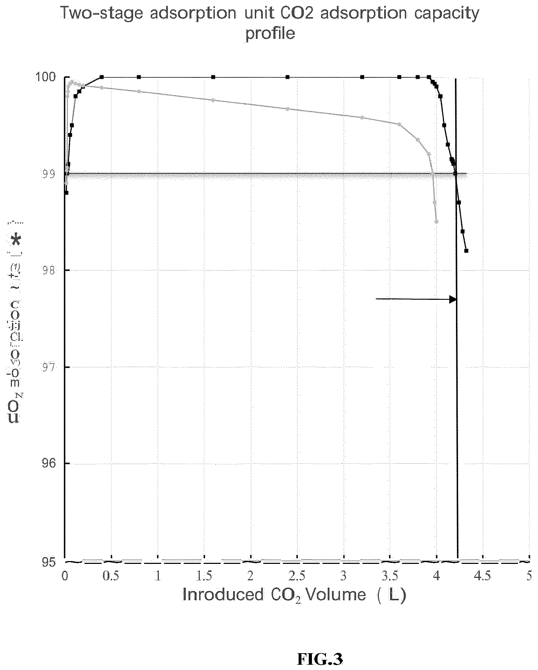

The CO2 adsorption capacity curves of the two-stage *in-situ* adsorption device (the solid-state adsorption unit and liquid-state adsorption unit) are shown in FIG. 3. In a characteristic curve of the CO2 adsorption capacity of the two-stage adsorption device, the abscissa indicates a volume of CO2 introduced (L), and the ordinate indicates a CO2 adsorption rate (%); a gas composition: 99.9% CO2, solid adsorption particles: 40 g of NaOH particles, and a liquid adsorption solution: 60 ml of NaOH at 3 mol/L were added for experiment. The results confirm that the adsorption efficiency and adsorption capacity of the device are significantly higher than those of the existing independent adsorption bottle and single-stage liquid *in-situ* adsorption device, which can effectively ensure a continuous progress of the biodegradation.

Specifically, in the aerobic fermentation unit 3, oxygen in the headspace is consumed gradually by microorganisms to conduct the aerobic composting; and carbon dioxide gas released by the microbial degradation is adsorbed by the adsorption unit, resulting in a negative pressure in the reactor. In order to maintain a balance of the gas pressure in the reactor, oxygen is continuously go through the respirometer cells unit 2 into the aerobic fermentation unit 3. Specifically, there is also a two-hole rubber sealing plug 35 for ensuring an airtight performance of the aerobic fermentation unit 3, and a threaded cap 36 is configured to fix the sealing plug of the aerobic fermentation unit 3.

In practical application, 100 g of a polylactic acid (PLA)-based degradable plastic bag was used as a test sample, and 100 g of cellulose was used as a reference material; a compost produced by a kitchen waste mechanical composting reactor as an inoculum, a moisture content of the compost was adjusted to 50%, and 600 g of the compost with an adjusted moisture content was injected into a 1 L fermentation reactor; and the fermentation reactor was placed in a constant-temperature water bath at 58°C for cultivation. The formed carbon dioxide can be captured by the two-stage adsorption module, thereby generating a decrease in pressure in the reactor. This drives oxygen gas through an ultra-low gas flow meter, allows for directly measuring the oxygen volume consumed by the microorganisms present in a certain sample. When the negative pressure in the fermentation reactor was gradually balanced by external oxygen gas, the oxygen supply unit gradually reduces oxygen supply. In such a cycle, after the composting test was conducted for more than 63 consecutive days usually, the cumulative release of carbon dioxide for 3 consecutive days was less than 1.5% of a total cumulative release of carbon dioxide; after reaching a preset test stop condition, the data processing unit automatically terminated the test, stopped test data acquisition, and calculated a biodegradation rate of each sample automatically.

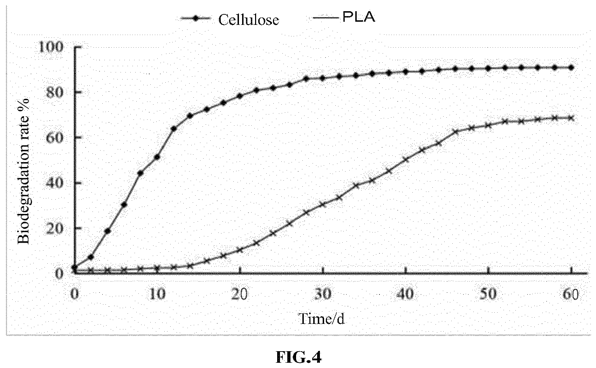

The specific test results were shown in FIG. 4. According to a working principle of the test system for aerobic biodegradability in the present disclosure, the test results of the degradation rates of cellulose and PLA were verified. By the system and method of the present disclosure to conduct experiments, a degradation effect of the cellulose had reached an expected effect, and a degradation effect of the PLA was slightly higher than the level of the prior art, and the test results had high repeatability.

In the example of the present disclosure, the test system for aerobic biodegradability has a high degree of automation, simple operation, and convenient use, and can process multiple samples, with a high test efficiency; oxygen enters the aerobic fermentation system through a gas flow meter to promote the degradation of the material, the carbon dioxide produced by the reaction is adsorbed, that causes the negative pressure in the reactor to ensure the external oxygen enter the reactor; the system improves the accuracy of data, and the automated processing greatly saves manpower.

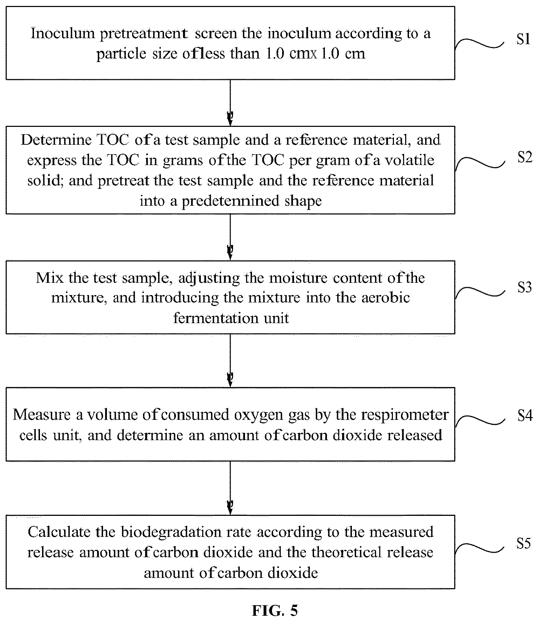

In this example, a test method for aerobic biodegradability is further provided, which can be applied to electronic devices, such as computers, mobile phones, and tablet computers. FIG. 5 shows a flowchart of the test method for aerobic biodegradability according to an example of the present disclosure. As shown in FIG. 5, the method includes the following steps:

S1, inoculum pretreatment: the inoculum is adjusted according to a particle size of less than 1.0 cm x 1.0 cm.

Specifically, the adjusting process needs to be kept uniform and free from large inert substances such as glass, stones, and metal pieces, and the inoculum is adjusted.

S2, TOC of a test sample and a reference material are determined, and the TOC is expressed in grams of the TOC per gram of a volatile solid; and the test sample and the reference material are pretreated into predetermined shape with maximum surface area of any individual piece that shall be about 1.5 cm x 1.5 cm.

S3, the test sample is mixed, the moisture content of the mixture is adjusted, and the mixture is introduced into the aerobic fermentation unit.

S4, a volume of consumed oxygen is measured by the respirometer cells unit, and an amount of carbon dioxide released is determined.

S5, the biodegradation rate of the degradation is calculated according to the measured release amount of carbon dioxide and the theoretical amount of evolved carbon dioxide; where the biodegradation rate represents the aerobic biodegradability.

In practical applications, the method of this example further included the following steps:

S01, oxygen from the oxygen supply unit was received, an oxygen concentration was determined, and a volume of consumed oxygen was calculated through the respirometer cells unit; reference was made to the corresponding steps of the system for a specific implementation process, which were not repeated in this example.

S02, aerobic organisms were degraded using the imported oxygen; reference was made to the corresponding steps of the system for a specific implementation process, which were not repeated in this example.

S03, a CO2 concentration produced by the degradation was calculated according to the volume of consumed oxygen and the oxygen concentration; reference was made to the corresponding steps of the system for a specific implementation process, which were not repeated in this example.

S04, a biodegradation rate of the degradation was calculated according to the CO2concentration; where the biodegradation rate represented the aerobic biodegradability. Reference was made to the corresponding steps of the system for a specific implementation process, which were not repeated in this example.

Specifically, in step S04, a process of calculating a biodegradation rate according to the CO2 concentration includes the following steps:

calculating a theoretical amount of evolved carbon dioxide (ThCO2) for the degradation at 0°C under a standard atmospheric pressure, expressed in g, according to the following formula:

$$ThCO2 = Mvs x Cvs x 44/12$$

in the formula:

Mvs indicates a volatile solid in a test sample added into a compost container at the beginning of a test, in g;
Cvs indicates a ratio ofTOC to the volatile solid in the test sample, in g/g;
44 and 12 indicate a relative molecular mass of carbon dioxide and a relative atomic mass of carbon;
Mvs is the volatile solid of biomass put into the fermentation reactor; compared with the total solid (TS), Mvs mainly characterizes the content of organic matter and helps better measure the degree of degradation of the sample;

calculating the biodegradation rate Dt(%) ofthe aerobic biodegradation according to the following formula:

$$Di = [(CO2)r''(CO2)e/ThCO2 \times 100$$

in the formula:

(CO2)r indicates an amount of carbon dioxide released equivalent to a cumulative consumption of oxygen by each compost fermentation reactor containing a mixing sample, in g;
(CO2)B indicates an average of carbon dioxide released equivalent to a cumulative consumption of oxygen by a blank fermentation reactor, in g;
ThCO2 indicates a theoretical amount of evolved carbon dioxide produced by the test sample, in g;
(CO2)r and (CO2)s represent the cumulative amount of carbon dioxide emitted by each compost container containing the mixing sample, and the average of the cumulative amount of carbon dioxide emitted by the blank container, respectively;

calculating a volume of accumulated gas through the respirometer cells unit:

$$Vs = l.fr,p''lw * VMc$$

in the formula:

Vs indicates an amount of carbon dioxide released equivalent to a volume of gas consumed by each reaction reactor at 0°C under a standard atmospheric pressure, in L;
frr indicates factors considering ambient temperature and air pressure;
$fw$ indicates a factor considering a water vapor content in the gas;
VMc indicates a theoretical volume of the cell chamber, which is the gas volume pass through under a certain flow rate with the chamber flipping once;

obtaining the following formula according to the ideal gas equation of state:

$$fr,p= Vg/Vgas = \{[Pgas *(Ts+20)]/(Ps *Tgas\}$$

according to the Dalton's Law of Partial Pressure and the Antoine equation, obtaining the factor (**L**) of the water vapor content in the gas:

$$Jw= 1-[101.32*10s.om1-mo.63!(233.426+ti)]1(160*PJ$$

calculating the amount of carbon dioxide (g) released by each compost fermentation reactor containing the mixing sample according to the following formula:

$$(CO2)T=44*(Vs)r/22.4$$

calculating the average amount of carbon dioxide (g) released from the blank fermentation reactor according to the following formula:

$$(CO2)B=44*(Vs)e/22.4$$

calculating the biodegradation rate (%) of the aerobic biodegradation according to the following formula:

$$Di=[Vsr-VsB\} Z:VThCO2 x 100$$

in the formula:

> *Vsr* indicates an amount of carbon dioxide released equivalent to a volume of gas consumed by each test sample-containing reaction reactor at 0°C under a standard atmospheric pressure, in L; and
> *VsB* indicates an average amount of carbon dioxide released equivalent to an average volume of gas consumed by each blank reaction reactor at 0°C under a standard atmospheric pressure, in L.

In the example of the present disclosure, the test method for aerobic biodegradability has a high degree of automation, simple operation, and convenient use, and can process multiple samples, with a high test efficiency; oxygen enters the aerobic fermentation system through a gas flow meter to promote the degradation of the material, the carbon dioxide produced by the reaction is adsorbed, that causes the negative pressure in the reactor to ensure the external oxygen enter the reactor; the method improves the accuracy of data, and the automated processing greatly saves manpower.

Embodiments of the present disclosure may take on various modifications and alterations without departing from the spirit and scope of the disclosure. Accordingly, it is to be understood that the embodiments of the present disclosure are not to be limited to the following described exemplary embodiments, but is to be controlled by the limitations set forth in the claims and any equivalents thereof

**Claims**

1. A test system for aerobic biodegradability, comprising: an oxygen supply unit, an aerobic fermentation unit, a respirometer cells unit, and a data processing unit, wherein

the oxygen supply unit is configured to store oxygen required for aerobic biodegradation;
the respirometer cells unit is connected to the aerobic fermentation unit through a gas inlet pipe, and is configured to introduce the oxygen from the oxygen supply unit to the aerobic fermentation unit; a one-way valve is provided on the gas inlet pipe to control a gas flow direction; the respirometer cells unit is further connected to the oxygen supply unit through a gas pipe, and is further configured to calculate a volume of consumed oxygen that is introduced into the aerobic fermentation unit due to a negative pressure;
the data processing unit is configured to acquire data during the aerobic biodegradation, and calculate a biodegradation rate of the aerobic biodegradation according to the volume of consumed oxygen;
the aerobic fermentation unit comprises a constant-temperature water bath, a fermentation reactor, and a two-stage *in-situ* adsorption module, and the fermentation reactor and the two-stage *in-situ* adsorption module are arranged in the constant-temperature water bath;
the fermentation reactor is configured to conduct degradation, and the degradation refers to that a reaction material is degraded by the aerobic biodegradation; and
the two-stage *in-situ* adsorption module is configured to adsorb carbon dioxide produced by the degradation of samples in the fermentation reactor.

2. The system according to claim 1, wherein the aerobic fermentation unit further comprises a stirring device, and the stirring device is a mechanical stirring bar or a magnetic stirrer.

3. The system according to claim 1, wherein the two-stage *in-situ* adsorption module comprises a solid-state adsorption unit and a liquid-state adsorption unit;

   the solid-state adsorption unit is arranged below an inner mouth of the fermentation reactor and fixed by a bottle mouth, and is configured to conduct primary adsorption of the carbon dioxide produced by the degradation; and
   the liquid-state adsorption unit is arranged above a mouth of the fermentation reactor and fixed by tightening with a threaded mouth, and is configured to adsorb the carbon dioxide not adsorbed by the solid-state adsorption unit.

4. The system according to claim 3, wherein

   the solid-state adsorption unit comprises a vented container, and a grid hole structure is provided on a side wall of the cavity to facilitate penetration of the carbon dioxide; and
   the liquid-state adsorption unit comprises an inverted-conical slope reflux structure outside a top cover, and the inverted-conical slope reflux structure is configured to return condensed water vapor in the fermentation reactor to the fermentation reactor.

5. The system according to claim 3, wherein the liquid-state adsorption unit comprises a double-layer cavity formed by an inverted-conical slope outside a top cover and the top cover, and a fluorinated coolant is injected into the double-layer cavity to prevent the loss of moisture in a material.

6. The system according to claim 3, wherein the liquid-state adsorption unit further comprises a top liquid injection hole and a bottom liquid discharge port; the top liquid injection hole is provided with a protruding column structure on a top cover surface, and is configured to inject an alkaline solution into the liquid-state adsorption unit; and the bottom liquid discharge port is arranged at a lowest point of a bottom level of the liquid-state adsorption unit, and is configured to drain the alkaline solution in the liquid-state adsorption unit.

7. The system according to claim 1, further comprising the data processing unit, wherein
   the data processing unit comprises a temperature sensor and a pressure sensor, and the temperature sensor and the pressure sensor are connected in the respirometer cells unit and are configured to conduct conversion into a volume of an oxygen-consuming gas under standard conditions.

8. A test method for aerobic biodegradability, applied to the test system for aerobic biodegradability according to any one of claims 1 to 7, and comprising the following steps:

   receiving oxygen from the oxygen supply unit, determining an oxygen concentration, and calculating a volume of consumed oxygen through the respirometer cells unit;
   conducting the degradation reaction of organic matter by the action of introduced oxygen and aerobic microorganisms;

calculating a CO2 concentration produced by the degradation according to the volume of consumed oxygen and the oxygen concentration; and

calculating a biodegradation rate according to the CO2 concentration; wherein the biodegradation rate represents the aerobic biodegradability.

9. The method according to claim 8, wherein a process of calculating a biodegradation rate according to the CO2 concentration comprises:

calculating a theoretical amount of evolved carbon dioxide *ThCO2* for the degradation at 0°C under a standard atmospheric pressure, expressed in g, according to the following formula:

$$ThCO2 = Mvs \times Cvs \times 44/12$$

in the formula:

*Mvs* indicates a volatile solid in a test sample added into a compost container at the beginning of a test, in g;
*Cvs* indicates a ratio of total organic carbon (TOC) to the volatile solid in the test sample, in g/g;
44 and 12 indicate a relative molecular mass of carbon dioxide and a relative atomic mass of carbon;
*Mvs* is the volatile solid of biomass put into the fermentation reactor; compared with the total solid (TS), *Mvs* mainly characterizes the content of organic matter and helps better measure the degree of degradation of the sample;

calculating the biodegradation rate Dt of the aerobic biodegradation according to the following formula:

$$Dt = [(CO2)r - (CO2)s]/ThCO2 \times 100$$

*(CO2)r* indicates an amount of carbon dioxide released equivalent to a cumulative consumption of oxygen by each compost fermentation reactor containing a mixing sample, in g;
*(CO2)s* indicates an average of carbon dioxide released equivalent to a cumulative consumption of oxygen by a blank fermentation reactor, in g;
*ThCO2* indicates a theoretical amount of evolved carbon dioxide produced by the test sample, mg;
*(Co2)r* and *(CO2)s* represent the cumulative amount of carbon dioxide emitted by each compost container containing the mixing sample, and the average of the cumulative amount of carbon dioxide emitted by the blank container, respectively;

calculating a volume of accumulated gas through the respirometer cells unit:

$$Vs = l.fr,p * fw * VMc$$

in the formula:

*Vs* indicates an amount of carbon dioxide released equivalent to a volume of gas consumed by each reaction reactor at 0°C under a standard atmospheric pressure, in L;
*frr* indicates factors considering ambient temperature and air pressure;
*Jw* indicates a factor considering a water vapor content in the gas;
$V_{MC}$ indicates a theoretical volume of the cell chamber, which is the gas volume pass through under a certain flow rate with the chamber flipping once;

obtaining the following formula according to the ideal gas equation of state:

$$fr,p = Vg/Vgas = \{[Pgas * (Ts+20)]/(Ps * Tgas\}$$

according to the Dalton's Law of Partial Pressure and the Antoine equation, obtaining the factor *Jw* of the water vapor content in the gas:

$$Jw = 1 - [101.32 * 1 os.o7m - 173o.63/(233.426 + ti)]/(760*PJ$$

calculating the amount of carbon dioxide released by each compost fermentation reactor containing the mixing sample according to the following formula:

$$(COz)y = 44*(Vs)T/22.4$$

calculating the average amount of carbon dioxide released from the blank fermentation reactor according to the following formula:

$$(CO2)B = 44*(Vs)B/22.4$$

calculating the biodegradation rate of the aerobic biodegradation according to the following formula:

$$Dt = [VST - VSB]*\frac{1}{2}\sqrt{ThCO2} 100$$

*Vsr* indicates an amount of carbon dioxide released equivalent to a volume of gas consumed by each test sample-containing reaction reactor at 0°C under a standard atmospheric pressure, in L; and
*Vss* indicates an average amount of carbon dioxide released equivalent to an average volume of gas consumed by each blank reaction reactor at 0°C under a standard atmospheric pressure, in L.

10. The method according to claim 8, further comprising the following steps:

S 1, inoculum pretreatment: screening the inoculum according to a particle size of less than 1.0 cm x 1.0 cm;
S2, determining TOC of a test sample and a reference material, and expressing the TOC in grams of the TOC per gram of a volatile solid; and pretreating the test sample and the reference material into a predetermined shape with maximum surface area of any individual piece that shall be about 1.5 cm x 1.5 cm;
S3, mixing the test sample, adjusting the moisture content of the mixture, and introducing the mixture into the aerobic fermentation unit;
S4, measuring a volume of consumed oxygen by the respirometer cells unit, and determining an amount of carbon dioxide released; and
S5, calculating the biodegradation rate according to the measured release amount of carbon dioxide and the theoretical amount of evolved carbon dioxide; wherein the biodegradation rate represents the aerobic biodegradability.

**FIG.1**

**FIG.2**

Two-stage adsorption unit CO2 adsorption capacity profile

FIG.3

FIG.4

Inoculum pretreatment screen the inoculum according to a particle size of less than 1.0 cm x 1.0 cm — S1

Determine TOC of a test sample and a reference material, and express the TOC in grams of the TOC per gram of a volatile solid; and pretreat the test sample and the reference material into a predetermined shape — S2

Mix the test sample, adjusting the moisture content of the mixture, and introducing the mixture into the aerobic fermentation unit — S3

Measure a volume of consumed oxygen gas by the respirometer cells unit, and determine an amount of carbon dioxide released — S4

Calculate the biodegradation rate according to the measured release amount of carbon dioxide and the theoretical release amount of carbon dioxide — S5

**FIG. 5**

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HUSSAIN FIDA ET AL: "Real-time biomonitoring of oxygen uptake rate and biochemical oxygen demand using a novel optical biogas respirometric system", JOURNAL OF ENVIRONMENTAL MANAGEMENT, ELSEVIER, AMSTERDAM, NL, vol. 277, 111467, 19 October 2020 (2020-10-19), pages 1-7, XP086361641, ISSN: 0301-4797, DOI: 10.1016/J.JENVMAN.2020.111467 [retrieved on 2020-10-19] * abstract * * page 1, right-hand column, paragraph 2 * * page 2, right-hand column, paragraph 3 - page 4, right-hand column, paragraph 3 * * figures 1,4 * | 1-10 | INV. G01N33/18 C12M1/34 C12M1/36 |
| Y | CN 111 471 584 A (YANG SHUAI) 31 July 2020 (2020-07-31) * abstract * * figure 1 * | 1-10 | |
| | -/-- | | TECHNICAL FIELDS SEARCHED (IPC) G01N C12M |

The present search report has been drawn up for all claims

| Place of search | Date of completion of the search | Examiner |
|---|---|---|
| The Hague | 14 December 2023 | Couteau, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

**EP 4 339 611 A1**

Europäisches Patentamt
European Patent Office
Office européen des brevets

## EUROPEAN SEARCH REPORT

Application Number

EP 23 19 0405

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ANONYMOUS: "ISO 14851:2019 Determination of the ultimate aerobic biodegradability of plastic materials in an aqueous medium – Method by measuring the oxygen demand in a closed respirometer", ISO NORM, ISO, CH, PAGE(S) 1 – 25 , 1 March 2019 (2019-03-01), XP009539937, Retrieved from the Internet: URL:https://www.iso.org/standard/70026.html * Section 1; page 1 * * Annex C * * Annex G * * figure C.1 * | 1-10 | |
| Y | RAGANATI FEDERICA ET AL: "Adsorption of Carbon Dioxide for Post-combustion Capture: A Review", ENERGY & FUELS, vol. 35, no. 16, 5 August 2021 (2021-08-05), pages 12845-12868, XP093111491, WASHINGTON, DC, US. ISSN: 0887-0624, DOI: 10.1021/acs.energyfuels.1c01618 * abstract * * page 12856, right-hand column, paragraph 2 * * figure 4 * | 1-10 | TECHNICAL FIELDS SEARCHED (IPC) |

-----

-/--

The present search report has been drawn up for all claims

| Place of search | Date of completion of the search | Examiner |
|---|---|---|
| The Hague | 14 December 2023 | Couteau, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

## EUROPEAN SEARCH REPORT

Application Number

**EP 23 19 0405**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SPANJERS H ET AL: "Respirometry", INTERNET CITATION, 31 December 2017 (2017-12-31), pages 133-176, XP002796730, Retrieved from the Internet: URL:https://experimentalmethods.org/wp-content/uploads/2017/12/Chapter-3.pdf [retrieved on 2020-01-08] * page 155, right-hand column, paragraph 4 * * figure 3.29 * ----- | 7 | |

TECHNICAL FIELDS
SEARCHED      (IPC)

The present search report has been drawn up for all claims

| Place of search | Date of completion of the search | Examiner |
|---|---|---|
| The Hague | 14 December 2023 | Couteau, Olivier |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 19 0405

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2023

| Patent document cited in search report | Publication date | Patent family member(s) | Publication date |
|---|---|---|---|
| CN 111471584 A | 31-07-2020 | NONE | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201721917449 X
- CN 202120574548